Europäisches Patentamt

European Patent Office

Office européen des brevets

(19)

(11) Veröffentlichungsnummer : **0 371 072 B1**

(12) **EUROPÄISCHE PATENTSCHRIFT**

(45) Veröffentlichungstag der Patentschrift :
24.04.91 Patentblatt 91/17

(51) Int. Cl.⁵ : **A61F 7/00**

(21) Anmeldenummer : 88907610.5

(22) Anmeldetag : 10.09.88

(86) Internationale Anmeldenummer :
PCT/DE88/00561

(87) Internationale Veröffentlichungsnummer :
WO 89/03201 20.04.89 Gazette 89/09

(54) **THERAPIEGERÄT ZUR KÄLTEAPPLIKATION.**

Die Akte enthält technische Angaben, die nach
dem Eingang der Anmeldung eingereicht
wurden und die nicht in dieser Patentschrift
enthalten sind.

(30) Priorität : 15.10.87 DE 8713858 U

(43) Veröffentlichungstag der Anmeldung :
06.06.90 Patentblatt 90/23

(45) Bekanntmachung des Hinweises auf die
Patenterteilung :
24.04.91 Patentblatt 91/17

(84) Benannte Vertragsstaaten :
AT BE CH DE FR GB IT LI LU NL SE

(56) Entgegenhaltungen :
EP-A-02 033 45
DE-C-33 054 34
US-A- 4 693 252
US-A-42 929 73

(73) Patentinhaber : WEBER, Dieter
Auf der Tödtheide 19
W-4800 Bielefeld 16 (DE)

(72) Erfinder : WEBER, Dieter
Auf der Tödtheide 19
W-4800 Bielefeld 16 (DE)

(74) Vertreter : Schirmer, Siegfried, Dipl.-Ing.
Patentanwalt Osningstrasse 10
W-4800 Bielefeld 1 (DE)

## Beschreibung

Die Erfindung betrifft ein Therapiegerät zur Kälteapplikation unter Abkühlung der Umgebungsluft, die über eine Lufteinlaßöffnung dem Gerät zuführbar und zielgerichtet als abgekühlte Luft über einen Isolierschlauch der Therapiestelle zuführbar ist.

Die Anwendung von Kälte in verschiedenen Formen zur Schmerzlinderung und Gewebeabschwellung ist ein seit langer Zeit bekanntes Therapieprinzip. Die Applikation kann hierbei durch feste, flüssige oder gasförmige Medien erfolgen.

Bei einer gasförmigen Applikation ist in der physikalischen Therapie die Anwendung von Stickstoffgasen bei Temperaturen um ca. –196°C nachweislich lindernd bei verschiedenen rheumatischen Erkrankungen und sportmedizinischen (stumpfen) Verletzungen bekannt. Der Vorteil dieser "Begasung" mit einem Stickstoffgemisch ist die extrem niedrige Temperatur, die gezielt in kürzester Zeit auch tiefere Gewebe bzw. Gelenkregionen erreichen kann.

Nachteilig bei der Stickstoffbegasungs-Methode ist u.a., daß ein Nachfüllen des Behälters mit flüssigem Stickstoff sehr häufig notwendig und zudem sehr kostspielig ist. Als Stand der Technik sind beispielsweise die europäische Patentanmeldung 0 203 345, die DE-OS 35 06 932 und die DE-PS 33 05 434 anzusehen.

Der Erfindung liegt die Aufgabe zugrunde, ein Therapiegerät zur Kälteapplikation so auszubilden, daß ein bestimmungsgemäßer Einsatz ohne Verwendung von flüssigem Stickstoff bei wesentlicher Reduzierung der Unterhaltskosten möglich ist.

Diese Aufgabe wird erfindungsgemäß durch ein ohne Verwendung von flüssigen Stickstoff elektrisch betriebenes und im Therapiegerät integriertes Kühlsystem mit Kühlaggregat und Kondensator gelöst. Vorteilhafterweise ist der Isolierschlauch mit einer auswechselbaren Aufsteckdüse versehen und am Gerätegehäuse ein Thermostatregelsystem angeordnet. Zur Anzeige einer Abweichung einer festgelegten Temperatur für die abströmende kalte Luft kann ein akustischer und/oder ein optischer Signalgeber angeordnet sein. Ebenso zweckmäßig ist zur Messung der Temperatur der Umluft und/oder der Temperatur am Applikationsort die Anordnung eines oder mehrerer Thermometer.

Zweckmäßige Ausgestaltungen der Erfindung sind in den übrigen Unteransprüchen aufgezeigt.

Durch das erfindungsgemäß ausgebildete Therapiegerät kann Umluft verschiedener Temperatur bis zu einer therapeuthisch nutzbaren Minustemperatur bei einfacher Bedienung und geringen Unterhaltskosten heruntergekühlt werden. Durch die richtige Wahl der auswechselbar angeordneten Steckdüse kann der therapeutischen Notwendigkeit entsprochen werden. Es ist außerdem möglich, der zu applizierenden kalten Luft andere Gase oder medizinische Zusätze beizumengen bzw. die kalte Luft anderweitig zu verändern.

Das erfindungsgemäße Gerät ist vorteilhaft vor allem bei der physikalischen Therpie in der Orthopädie, Rheumatologie und Sportmedizin sowie der Veterinärmedizin einsetzbar.

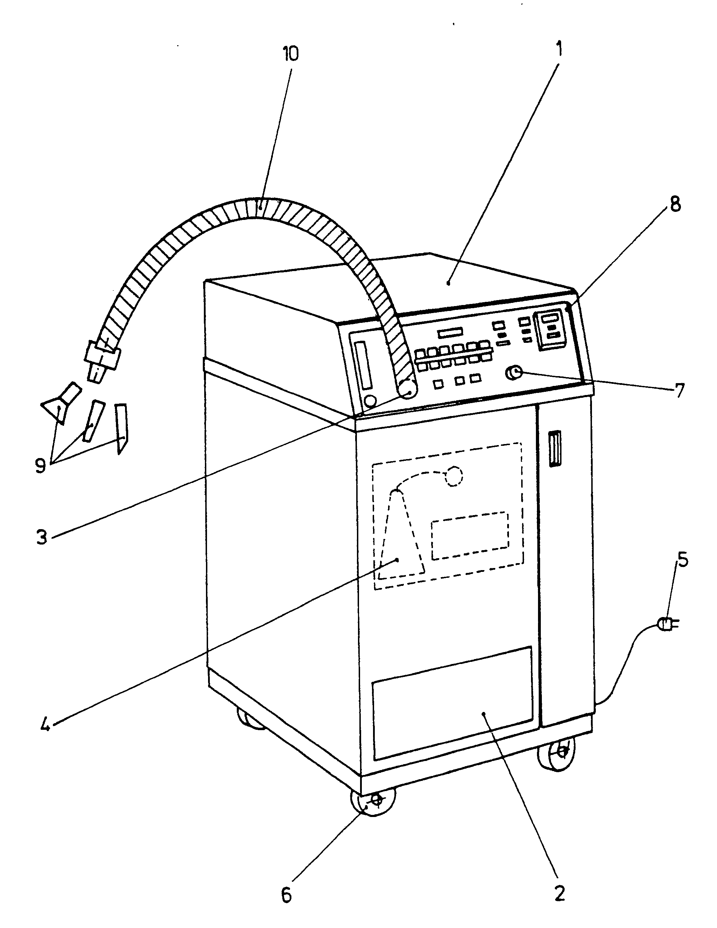

Ein Ausführungsbeispiel der Erfindung ist in der Zeichnung dargestellt und wird im folgenden näher beschrieben. Die einzige Figur zeigt schematisch eine isometrische Darstellung eines Therapiegeräts.

Im Therapiegerät 1 ist ein integriertes Kühlsystem 4 angeordnet. Über die Lufteinlaßöffnung 2 wird dem Therapiegerät 1 Umluft zugeführt. Die heruntergekühlte luft wird über den Luftauslaß 3 und einen angeschlossenen Isolierschlauch 10 an den Applikationsort des Patienten geführt. Das freie Ende des Isolierschlauchs 10 ist mit einer entsprechend den jeweiligen Bedingungen ausgebildeten Aufsteckdüse 9 versehen. Auf der Vorderseite des Therapiegeräts 1 sind ein Thermostatregelsystem 7 und ein Thermometer 8 angeordnet. Die Stromversorgung erfolgt mittels Netzstecker 5 vom Stromnetz aus. Zum leichteren Transport des Therapiegeräts 1 sind auf der Unterseite Fahrrollen 6 angeordnet,

## Ansprüche

1. Therapiegerät (1) zur Kälteapplikation unter Abkühlung der umgebungsluft die über eine Lufteinlaßöffnung (2) dem Gerät zuführbar und zielgerichtet als abgekühlte Luft über einen Isolierschlauch (10) der Therapiestelle zuführbar ist, gekennzeichnet durch ein ohne Verwendung von flüssigem Stickstoff elektrisch betriebenes und im Therapiegerät (1) integriertes Kühlsystem (4) mit Kühlaggregat und kondensator.

2. Therapiegerät nach Anspruch 1, dadurch gekennzeichnet, daß der Isolierschlauch (10) mit einer auswechselbaren Aufsteckdüse (9) versehen ist.

3. Therapiegerät nach Anspruch 1 oder 2, dadurch gekennzeichnet, daß am Gerätegehäuse ein Thermostatregelsystem (7) angeordnet ist.

4. Therapiegerät nach einem der Ansprüche 1 bis 3, dadurch gekennzeichnet, daß zur Anzeige einer Abweichung einer festgelegten Temperatur für die abströmende kalte Luft ein akustischer und/oder ein optischer Signalgeber angeordnet ist.

5. Therapiegerät nach einem der Ansprüche 1 bis 4, dadurch gekennzeichnet, daß zur Messung der Temperatur der Umluft und/oder der Temperatur am Applikationsort ein oder mehrere Thermometer (8) angeordnet ist bzm. sind.

6. Therapiegerät nach einem der Ansprüche 1 bis 5, dadurch gekennzeichnet, daß dem Therapiegerät (1) zum Transport direkt oder indirekt Fahrrollen (6) zugeordnet sind.

7. Therapiegerät nach einem der Ansprüche 1 bis 6, dadurch gekennzeichnet, daß als Stromquelle eine Batterie zugeordnet ist.

8. Therapiegerät nach einem der Ansprüche 1 bis 7, dadurch gekennzeichnet, daß zu stufenlosen Veränderung der Ausströmgeschwindigkeit der kalten Luft ein Lüfter angeordnet ist.

9. Therapiegerät nach Anspruch 8, dadurch gekennzeichnet, daß ein Ventilator angeordnet ist.

**Claims**

1. Therapy appliance (1) for the application of coldness while cooling down the ambient air which is feedable to the appliance by way of an air inlet opening (2) and feedable in aimed manner as cooled air by way of an insulating hose (10) to the therapy place, characterised by a cooling system (4), which is integrated in the therapy appliance (1) and electrically operated without the use of liquid nitrogen, with cooling unit and condenser.

2. Therapy appliance according to claim 1, characterised thereby, that the insulating hose (10) is provided with an exchangeable attachment nozzle (9).

3. Therapy appliance according to claim 1 or 2, characterised thereby, that a thermostatic regulating system (7) is arranged at the appliance housing.

4. Therapy appliance according to one of the claims 1 to 3, characterised thereby, that an acoustic and/or an optical signal transmitter is arranged for the indication of a departure from a fixed temperature for the outflowing cold air.

5. Therapy appliance according to one of the claims 1 to 4, characterised thereby, that one or more thermometers (8) is or are arranged for the measurement of the temperature of the ambient air and/or of the temperature at the place of application.

6. Therapy appliance according to one of the claims 1 to 5, characterised thereby, that castors (6) are directly or indirectly associated with the therapy appliance (1) for transport.

7. Therapy appliance according to one of the claims 1 to 6, characterised thereby, that a battery is associated as current source.

8. Therapy appliance according to one of the claims 1 to 7, characterised thereby, that a fan is arranged for the stepless variation of the outflow speed of the cold air.

9. Therapy appliance according to claim 8, characterised thereby, that a ventilator is arranged.

être introduit dans l'appareil par une ouverture d'admission d'air (2) et emmené au but, comme air refroidi, à l'endroit de la thérapie par un tuyau isolant (10), caractérisé en ce qu'il comprend un système de refroidissement (4) avec groupe frigorifique et condensateur, sans utilisation d'azote liquide, fonctionnant électriquement et intégré dans l'appareil thérapeutique (1).

2. Appareil thérapeutique selon la revendication 1, caractérisé en ce que le tuyau isolant (10) est muni d'une buse (9) emboîtable et remplaçable.

3. Appareil thérapeutique selon une des revendications 1 ou 2, caractérisé en ce que sur le boîtier de l'appareil est disposé un système de réglage thermostatique (7).

4. Appareil thérapeutique selon une des revendications 1 à 3, caractérisé en ce que pour indiquer une déviation d'une température fixée pour le flux d'air froid, est installé un transmetteur de signal acoustique et/ou optique.

5. Appareil thérapeutique selon une des revendications 1 à 4, caractérisé en ce que pour mesurer la température de l'air ambiant et/ou la température à l'endroit d'application, est ou sont mis en place un ou plusieurs thermomètres (8).

6. Appareil thérapeutique selon une des revendications 1 à 5, caractérisé en ce que pour le transport de l'appareil thérapeutique (1) sont associées directement ou indirectement des roulettes (6).

7. Appareil thérapeutique selon une des revendications 1 à 6, caractérisé en ce que comme source de courant électrique, est associée une batterie.

8. Appareil thérapeutique selon une des revendications 1 à 7, caractérisé en ce que pour une variation progressive de la vitesse du courant de sortie d'air froid, est disposée une soufflante.

9. Appareil thérapeutique selon la revendication 8, caractérisé en ce qu'un ventilateur est mis en place.

**Revendications**

1. Appareil thérapeutique (1) pour application de froid, par refroidissement de l'air ambiant, qui peut